# EUROPÄISCHE PATENTSCHRIFT

(11) **EP 0 796 833 B1**
(45) Veröffentlichungstag und Bekanntmachung des Hinweises auf die Patenterteilung: **05.09.2001**
(21) Anmeldenummer: 97104443.3
(22) Anmeldetag: 15.03.1997
(51) Int. Cl.: C07C 37/08, C07C 39/225, C07C 409/08, C07C 409/10, C07C 409/12

(54) **Verfahren zur Herstellung von beta-Naphthol**
Process for the preparation of beta-naphthol
Procédé pour la préparation de beta-naphthol

(30) Priorität: 20.03.1996 PL 31341996
(43) Veröffentlichungstag der Anmeldung: 24.09.1997
(73) Patentinhaber: Rütgers Kureha Solvents GmbH, 47138 Duisburg (DE)
(72) Erfinder: Stec, Zbigniew, 44-100 Gliwice (PL); Zawadiak, Jan, 44-105 Gliwice (PL); Knips, Ulrich, 59174 Kamen (DE); Zellerhoff, Robert, 46499 Hamminkeln (DE); Gilner, Danuta, 44-101 Gliwice (PL); Orlinska, Beata, 40-868 Katowice (PL); Polaczek, Jerzy, 02-784 Warszawa (PL); Tecza, Witold, 05-090 Raszyn (PL); Machowska, Zofia, Warszawa (PL)
(74) Vertreter: Cohausz & Florack

(56) Entgegenhaltungen:
- EP-A- 0 308 133
- EP-A- 0 370 729
- GB-A- 610 293
- GB-A- 626 095
- GB-A- 641 250
- GB-A- 654 035
- GB-A- 754 862
- GB-A- 757 164
- GB-A- 760 367
- GB-A- 1 284 326
- GB-A- 1 496 227
- US-A- 2 751 418
- US-A- 3 939 211

## Beschreibung

Die Erfindung betrifft ein Verfahren zur Herstellung von β-Naphthol durch katalytische Oxidation von 2-Isopropylnaphthalin mit Sauerstoff und Zersetzung des so gebildeten Hydroperoxids.

Ein entsprechendes Verfahren ist in der folgenden Reaktionsgleichung beispielhaft für die Herstellung von β-Naphthol dargestellt.

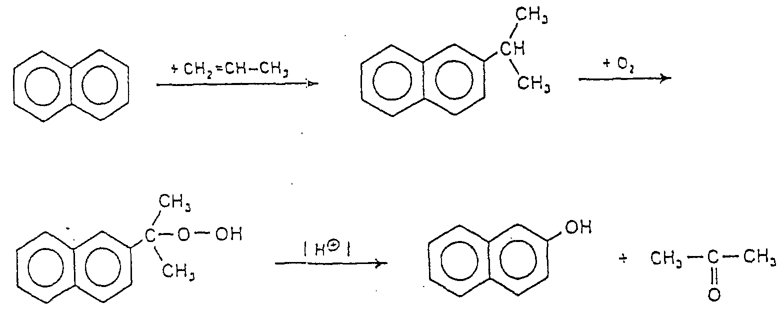

Dieses Verfahren, welches beschrieben ist in H.G. Franck, J.W. Stadelhofer, Industrielle Aromatenchemie, Springer Verlag 1987, läßt sich im industriellen Maßstab jedoch nicht durchführen, da bei der Oxidation von 2-Isopropylnaphthalin (2-IPN) nur eine sehr geringe Umsatzrate erreicht wird, so daß letztendlich der maximal erreichte Hydroperoxid-Gehalt 30 % nicht überschreitet.

Ein weiteres Hindernis bei der Durchführung dieses Verfahrens ist die Beschaffung eines technischen Rohstoffs als Ausgangsmaterial, der frei von oder nur wenig 1-Isopropylnaphthalin enthält. Dieses beeinträchtigt die Umsetzung von 2-Isopropylnaphthalin zu dem Hydroperoxid des 2-Isopropylnaphthalins. Wenn das Ausgangsmaterial beispielsweise 10 % 1-IPN enthält, beträgt die maximale Hydroperoxid-2-IPN-Konzentration im Produkt sogar nur 20 %.

Die US 3 939 211 beschreibt ein Verfahren zur Oxidation von 2-Isopropylnaphthalin mit molekularem Sauerstoff unter Verwendung eines Nickel-(II)Komplexes als Katalysator mit anschließender saurer Hydrolyse zu β-Naphthol, wobei ein möglichst reines 2-Isopropylnaphthalin als Ausgangsmaterial eingesetzt werden muß, um zufriedenstellende Ausbeuten zu erhalten. Die EP 0 308 133 Al offenbart ein Verfahren zur Oxidation von alkylsubstituierten Naphthalenen zum Hydroperoxid mit molekularem Sauerstoff in wässriger, alkalischer Emulsion in Gegenwart eines methylenhaltigen aromatischen Kohlenwasserstoffs als Katalysator. Die EP 0 370 729 Al beschreibt ein Verfahren zur Oxidation von Diisopropylnaphthalin mit molekularem Sauerstoff in wässriger, alkalischer Emulsion zum Hydroperoxid und anschließender saurer Hydrolyse zu Isopropylnaphthol. Die GB 654 035 beschreibt ein Oxidationsverfahren, das ohne Zusatz von Katalysatoren durchgeführt wird. Zum Erhalt akzeptabler Ausbeuten muß 2-Isopropylnaphthalin einer Reinheit von mindestens 95% eingesetzt werden. Selbst bei Einsatz von 98% 2-Isopropylnapthalin wird nur eine Hydroperoxid-Ausbeute von 46% erreicht. Die GB 757 164 beschreibt ein Verfahren zur Oxidation von 2-Isopropylnaphthalin in Gegenwart von 1-Isopropylnaphthalin zu 2-Isopropylnaphthalin-Hydroperoxid mit Luft-Sauerstoff in alkalischer Dispersion ohne Zusatz von weiteren Katalysatoren. Um den störenden Einfluß von 1-Isopropylnaphthalin zu vermeiden wird vorgeschlagen, die Oxidation nur bis zu einer Hydroperoxid-Ausbeute von 30 bis 45% durchzuführen und das nicht umgesetzte Ausgangsmaterial durch Kristallisation zurückzugewinnen und erneut zu oxidieren. Die GB 641 250 beschreibt ein Verfahren zur Oxidation von Diisopropylbenzol mit molekularem Sauerstoff in wässriger alkalischer Dispersion ohne Zusatz von Katalysatoren zu Diisopropylhydroperoxid.

Der Erfindung liegt die Aufgabe zugrunde, ein Verfahren bereitzustellen, welches geeignet ist, 1- und 2-Isopropylnaphthalin enthaltende Prozeßströme ohne Vorreinigung derselben zur Herstellung von β-Naphthol einzusetzen.

Diese Aufgabe wird gelöst durch ein Verfahren zur Herstellung von β-Naphthol durch katalytische Oxidation von 2-Isopropylnaphtalin mit Sauerstoff und Zersetzung des so gebildeten Hydroperoxids zu β-Naphtol, wobei das 2-Isopropylnaphtalin mit einer wäßrigen Katalysatorlösung, die Kupferverbindungen, anorganische Cyanide oder organische Nitrile enthält, emulgiert, auf eine Temperatur von 50°C bis zur Siedetemperatur der Emulsion erwärmt, zwei bis zwanzig Stunden lang der Einwirkung von Sauerstoff ausgesetzt und das entstandene Hydroperoxid in Gegenwart: einer anorganischen Säure als Katalysator in β-Naphthol und Aceton gespalten wird.

Überraschend hat sich gezeigt, daß durch das erfindungsgemäße Verfahren beispielsweise eine Umsetzung von 2-Isopropylnaphthalin zum Hydroperoxid von mehr als 50 % in einer Reaktionsstufe möglich ist, selbst wenn der technische Rohstoff 8 bis 11% 1-Isopropylnaphthalin enthält. Nach dem Zerfall des Hydroperoxids beträgt die β-Naphthol-Ausbeute 86%.

Als Katalysator werden vorzugsweise anorganische oder organische Kupferverbindungen, anorganische Cyanide oder organische Nitrile eingesetzt, die die Oxidation aktivieren. Besonders bevorzugte Kupferverbindungen sind die Chloride, Stearate, Acetate, Carbonate, Acetylacetone, Bromide und Kupfer(I)- und Kupfer(II)-oxide. Diese Kupferverbindungen werden vorzugsweise in einer Menge von 0,000001 bis 0,01 Mol je Mol 2-Isopropylnaphthalin eingesetzt. Besonders bevorzugte anorganische Cyanide und organische Nitrile sind Natrium-, Kalium- und Ammoniumcyanid, Acetonitril, Chloracetonitril, Benzonitril, Azobiscyanocyclohexan, Azobisisobutyronitril und Tetracyanoethylen eingesetzt. Diese werden vorzugsweise in einer Menge von 0,00001 bis 0,2 Mol je Mol 2-Isopropylnaphthalin verwendet.

Gemäß einer erfindungsgemäß bevorzugten Ausführungsform können Alkalihydroxide oder Alkalimetallcarbonate in einer Menge von 0,001 bis 1 Masse %, bezogen auf die Masse der Katalysatorlösung. Vorzugsweise steht die Kohlenwasserstoffphase mit der wäßrigen Katalysatorlösung in einem Volumenverhältnis von 1 : 5 bis 3 : 1, gemäß einer besonders bevorzugten Ausführungsform erfolgt die Emulgierung aber im Verhältnis 1 : 1.

Nach abgeschlossener Oxidation wird die organische Phase von der wäßrigen Phase getrennt, mit Aceton verdünnt, beispielsweise 10 bis 200 Gewichtsteile Aceton, und das darin enthaltene 2-Isopropylnaphthalinhydroperoxid in Gegenwart einer anorganischen Säure, beispielsweise Schwefelsäure, durch Erwärmen auf eine Temperatur von 20 bis 70°C gespalten. Diese Reaktion wird vorzugsweise bei der Siedetemperatur von Aceton durchgeführt, indem die organische Phase in die Acetonlösung der anorganischen Säure eingeleitet wird. Das so gebildete β-Naphthol läßt sich mittels Extraktion mit alkalischen Lösungen oder mittels Kristallisation bzw. Destillation leicht ausscheiden, und die Kohlenwasserstoffschicht wird nach der Extraktion zusätzlich mit Wasserstoffperoxid in Gegenwart einer anorganischen Säure als Katalysator oxidiert, wonach die zusätzlich erhaltenen Hydroperoxide erneut gespalten werden.

Die Herstellung von β-Naphthol nach den erfindungsgemäßen Verfahren hat im Vergleich zu bereits bekannten Verfahren viele Vorteile. Die erfindungsgemäße Verfahrensweise ermöglicht insbesondere eine Umsetzung des im Ausgangsmaterial enthaltenen 2-Isopropylnaphthalins zum Hydroperoxid von 50 % in einem Durchgang und durch die zusätzliche Behandlung des Gemischs nach dem Ausscheiden des β-Naphthols mit Wasserstoffperoxid läßt sich diese Umsetzung in einem Durchgang auf 60 bis 70 % erhöhen.

Die folgenden Beispiele erläutern die Erfindung.

### Beispiel I

In einem thermostatischen Glasreaktor mit magnetischem Rührwerk werden 3 g technischen 2-Isopropylnaphthalins (Rütgers Kureha Solvents GmbH, Duisburg, Deutschland) Reinheit 90,2 %; 1-Isopropylnaphthalininhalt 9,0 %, 3 cm³ 0,3-prozentiger wäßriger NaOH-Lösung, 0,001 g Palmitinsäure und 0,0005 g Kupfercyanid geleitet; all dies wird 15 Minuten lang bei Zimmertemperatur bis zur Emulsions-bildung gemischt und dann auf 90°C erwärmt. Darauf wird technischer Sauerstoff zugeführt und die Emulsion 13 Stunden lang bei dieser Temperatur erhitzt. Nach Abscheiden der wäßr:Lgen Schicht mittels einer Zentrifuge enthält die organische Schicht 54,3 % 2-Isopropylnaphthalinhydroperoxid und 9,3 % 2-Isopropylnaphthylalkohol.

Diese Schicht wird dann in siedende einprozentige Schwefelsäurelösung in Aceton eingetröpfelt, bei gleichzeitigem Abscheiden des Acetons, das sich während der Zersetzung des Hydroperoxids gebildet hat. Nach der Extraktion des Destillationsrückstands mit 15 %iger Natriumhydroxidlösung und der Sättigung des Extrakts mit 20 %iger Schwefelsäurelösung erhält man 1,18 g β-Naphthol, welches nach einmaligem Umkristallisieren aus verdünntem Ethanol bei einer Temperatur von 121,9°C schmilzt. In die organische Schicht werden nach der Extraktion 3 cm³ 30 %iger Wasserstoffperoxidlösung und Schwefelsäure zugegeben, wodurch man weiteres 2-Isopropylnaphthalinhydroperoxid erhält, welches nach dessen Zersetzung (wie oben) zusätzlich 0,16 g β-Naphthol ergibt.

### Beispiele II bis IX

Auf gleiche Weise wie in Beispiel I und unter Anwendung der in Tafel 1 angeführten Katalysatoren, Aktivatoren und Prozessbedingungen erlangt man die in Tafel 1 erwähnten Konversionen des 2-Isopropylnaphthalins zum Hydroperoxid technischen 2-Isopropylnaphthalins, dessen Charakteristik aus Beispiel I zu ersehen ist. In allen diesen Fällen erhält man 1,03 bis 1,16 g β-Naphthol und zusätzlich noch etwa 0,13 bis 0,17 g β-Naphthol nach Zersetzung des während der Oxidation des 2-Isopropylnaphthylalkohols mit Wasserstoffperoxid entstandenen Hydroperoxids.

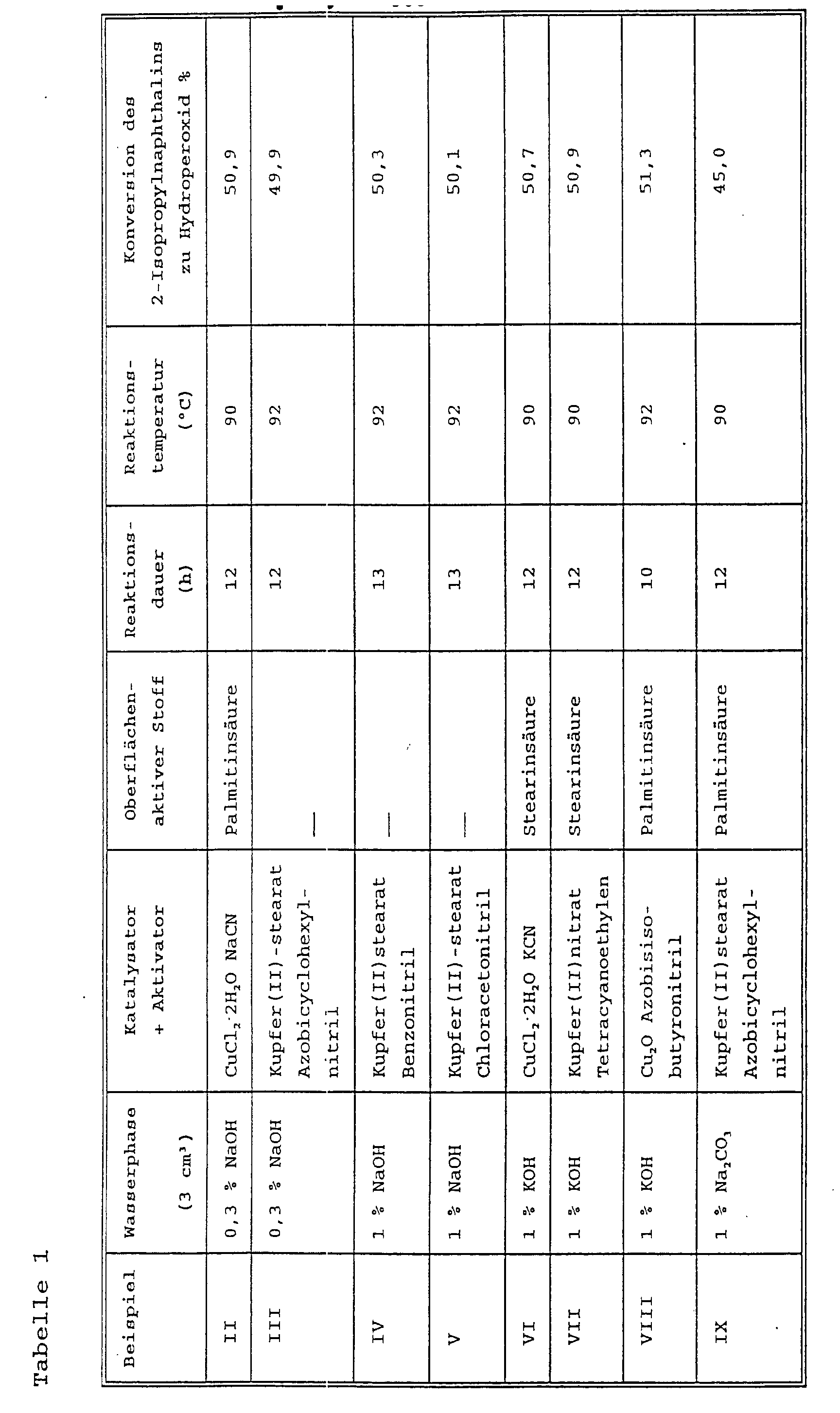

## Patentansprüche

1. Verfahren zur Herstellung von β-Naphthol durch katalytische Oxidation von 2-Isopropylnaphtalin mit Sauerstoff und Zersetzung des so gebildeten Hydroperoxids, **dadurch gekennzeichnet,** daß das 2-Isopropylnaphthalin mit einer wäßrigen Katalysatorlösung, die Kupferverbindungen, anorganische Cyanide oder organische Nitrile enthält, emulgiert, auf eine Temperatur von 50 °C bis zur Siedetemperatur der Emulsion erwärmt, zwei bis zwanzig Stunden lang der Einwirkung von Sauerstoff ausgesetzt und das entstandene Hydroperoxid in Gegenwart einer anorganischen Säure als Katalysator in β-Naphthol und Aceton gespalten wird.

2. Verfahren nach Anspruch 1, **dadurch gekennzeichnet,** daß die Kupferverbindungen in einer Menge von 0,000001 bis 0,01 Mol je Mol 2-Isopropylnaphthalin eingesetzt werden.

3. Verfahren nach Anspruch 1, **dadurch gekennzeichnet,** daß die anorganischen Cyanide oder organischen Nitrile in einer Menge von 0,00001 bis 0,2 Mol je Mol 2-Isopropylnaphthalin eingesetzt werden.

4. Verfahren nach einem der Ansprüche 1 bis 3, **dadurch gekennzeichnet**, daß als Katalysator Kupfercyanid in einer Menge von 0,000001 bis 0,01 Mol je Mol Isoalkylaromat eingesetzt wird.

5. Verfahren nach einem der Ansprüche 1 bis 4, **dadurch gekennzeichnet**, daß der wäßrigen Katalysatorlösung Alkalihydroxide oder Alkalicarbonate in einer Menge von 0,001 bis 1 Gewichtsteilen je 100 Gewichtsteile Katalysatorlösung zugesetzt werden.

6. Verfahren nach einem der Ansprüche 1 bis 5, **dadurch gekennzeichnet,** daß oberflächenaktive Substanzen, insbesondere Fettsäuren oder Fettsäuresalze, in einer Menge von 0,0001 bis 0,1 Gewichtsteilen je 100 Gewichtsteile Katalysatorlösung zugemischt werden.

7. Verfahren nach einem der Ansprüche 1 bis 6, **dadurch gekennzeichnet**, daß das 2-Isopropylnaphthalin mit 1 wäßriger Katalysatorlösung im Volumenverhältnis 1:1 emulgiert wird.

## Claims

1. Process for the production of β-naphthol by catalytic oxidation of 2-isopropyl naphthalene with oxygen and decomposition of the hydroperoxide thus formed, **characterized in that** 2-isopropyl naphthalene is emulsified with an aqueous catalyst solution, comprising copper compounds, inorganic cyanides or organic nitriles, warmed to a temperature ranging from 50°C up to the boiling temperature of the emulsion, exposed for 2 to 20 hours to the action of oxygen, and the resultant hydroperoxide is cleaved into β-naphtol and acetone in the presence of an inorganic acid as catalyst.

2. Process according to claim 1, **characterized in that** the copper compounds are used in an amount ranging from 0.000001 to 0.01 mole per mole 2-isopropyl naphthalene.

3. Process according to claim 1, **characterized in that** the inorganic cyanide or organic nitriles are used in an amount ranging from 0.00001 to 0.2 mole per mole 2-isopropyl naphthalene.

4. Process according to one of the claims 1 to 3, **characterized in that** as catalyst copper cyanide is used in an amount ranging from 0.00001 to 0.01 mole per mole isoalkyl aromatic substance.

5. Process according to one of the claims 1 to 4, **characterized in that** the aqueous catalyst solution contains alkali hydroxides or alkali carbonates in an amount ranging from 0.001 to 1 part by weight per 100 parts by weight of catalyst solution.

6. Process according to one of the claims 1 to 5, **characterized in that** surface active substances, in particular fatty acids or fatty acid salts are admixed in an amount ranging from 0.0001 to 0.1 part by weight per 100 parts by weight of catalyst solution.

7. Process according to one of the claims 1 to 6, **characterized in that** the 2-isopropyl naphthalene is emulsified with an aqueous catalyst solution in the volume ratio of 1:1.

## Revendications

1. Procédé de préparation de β-naphtol par oxydation catalytique du 2-isopropylnaphtalène avec de l'oxygène et décomposition de l'hydroperoxyde ainsi formé, **caractérisé en ce que** le 2-isopropylnaphtalène est émulsionné avec une solution aqueuse de catalyseur qui contient un composé du cuivre, un cyanure inorganique ou un nitrile organique, chauffé jusqu'à une température allant de 50°C à la température d'ébullition de l'émulsion, exposé pendant deux à vingt heures à l'action de l'oxygène et l'hydroperoxyde formé est clivé en présence d'un acide inorganique comme catalyseur, en β-naphtol et acétone.

2. Procédé suivant la revendication 1, **caractérisé en ce que** les composés du cuivre sont mis en oeuvre en une quantité allant de 0,000001 à 0,01 mole par mole de 2-isopropylnaphtalène.

3. Procédé suivant la revendication 1, **caractérisé en ce que** les cyanures inorganiques ou les nitriles organiques sont mis en oeuvre en une quantité allant de 0,00001 à 0,2 mole par mole de 2-isopropylnaphtalène.

4. Procédé suivant l'une quelconque des revendications 1 à 3, **caractérisé en ce que** l'on met en oeuvre comme catalyseur, le cyanure de cuivre en une quantité allant de 0,000001 à 0,01 mole par mole d'aromatique d'isoalcoyle.

5. Procédé suivant l'une quelconque des revendications 1 à 4, **caractérisé en ce que** la solution aqueuse de catalyseur est ajoutée à l'hydroxyde alcalin ou au carbonate alcalin en une quantité allant de 0,001 à 1 partie en poids par 100 parties en poids de la solution de catalyseur.

6. Procédé suivant l'une quelconque des revendications 1 à 5, **caractérisé en ce que** l'on mélange des substances tensioactives, en particulier des acides gras ou sels d'acide gras, en une quantité allant de 0,0001 à 0,1 partie en poids par 100 parties en poids de la solution de catalyseur.

7. Procédé suivant l'une quelconque des revendications 1 à 6, **caractérisé en ce que** le 2-isopropylnaphtalène est émulsionné avec la solution aqueuse de catalyseur suivant un rapport volumique de 1:1.
